(19) **Europäisches Patentamt / European Patent Office / Office européen des brevets**

(11) **EP 4 710 963 A1**

(12) **EUROPEAN PATENT APPLICATION**
published in accordance with Art. 153(4) EPC

(43) Date of publication:
18.03.2026 Bulletin 2026/12

(21) Application number: 24803522.2

(22) Date of filing: 09.05.2024

(51) International Patent Classification (IPC):
$A61L\ 31/12^{(2006.01)}$    $A61L\ 27/20^{(2006.01)}$
$A61L\ 27/22^{(2006.01)}$    $A61L\ 27/26^{(2006.01)}$
$A61L\ 27/48^{(2006.01)}$    $A61L\ 27/58^{(2006.01)}$
$A61L\ 31/04^{(2006.01)}$    $A61L\ 31/06^{(2006.01)}$
$A61L\ 31/14^{(2006.01)}$    $C08J\ 5/18^{(2006.01)}$
$C08L\ 67/04^{(2006.01)}$    $C08L\ 101/00^{(2006.01)}$
$C08L\ 101/16^{(2006.01)}$

(52) Cooperative Patent Classification (CPC):
A61L 27/20; A61L 27/22; A61L 27/26; A61L 27/48;
A61L 27/58; A61L 31/04; A61L 31/06; A61L 31/12;
A61L 31/14; C08J 5/18; C08L 67/04; C08L 101/00;
C08L 101/16

(86) International application number:
PCT/JP2024/017274

(87) International publication number:
WO 2024/232413 (14.11.2024 Gazette 2024/46)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 09.05.2023 JP 2023077268

(71) Applicant: Kureha Corporation
Chuo-ku
Tokyo 103-8552 (JP)

(72) Inventors:
• SHIMADA, Hironao
  Tokyo 103-8552 (JP)
• SEKINE, Fujio
  Tokyo 103-8552 (JP)
• YAMASHITA, Yusuke
  Tokyo 103-8552 (JP)
• OHIRA, Yurika
  Tokyo 103-8552 (JP)
• TAKANOHASHI, Masato
  Tokyo 103-8552 (JP)

(74) Representative: Hoefer & Partner Patentanwälte
mbB
Pilgersheimer Straße 20
81543 München (DE)

(54) **MEDICAL FILM AND PRODUCTION METHOD THEREFOR**

(57) Provided is a medical film containing particles containing polyglycolic acid, in which the particles have good dispersibility and are prevented from aggregation. The medical film according to an embodiment of the present invention contains: a crosslinked polymer material; and PGA dispersoids that are particles containing a polymer or aggregates of the particles, the polymer having a ratio of a constituent unit derived from glycolic acid of 40 mass% or more. The content of the PGA dispersoids per 100 parts by weight of the crosslinked polymer material is 2 parts by weight or more and 40 parts by weight or less, and a coefficient of variation CV of the number of particles contained in rectangular regions is 0.6 or less, where the coefficient of variation CV is determined from the number of particles measured in each of 16 rectangular regions with a size of 5 mm x 4 mm set so as not to overlap with each other.

EP 4 710 963 A1

FIG. 1A

FIG. 1B

**Description**

**[TECHNICAL FIELD]**

The present invention relates to a medical film and a method for producing the medical film.

**[BACKGROUND ART]**

Medical films (adhesion barrier materials) for preventing adhesion between organs are known. Such a film is attached to an organ after surgery to prevent adhesion between the organ and another organ when a wound is healing. For the adhesion barrier material, a bioabsorbable material, such as a crosslinked product of hyaluronic acid and carbomethyl cellulose, is used.

A medical film containing a crosslinked polymer material and a disintegration retardant containing a biodegradable polyester, such as polyglycolic acid (PGA), can retain its shape in a living body for a long period (Patent Document 1). Patent Document 1 describes that the disintegration retardant releases an acid to suppress the disintegration of the shape of the medical film. In addition, Patent Document 1 describes that PGA with a lower molecular weight has higher acid-releasing ability.

[Citation List]

[Patent Document]

Patent Document 1: WO 2021/201150

**[SUMMARY OF INVENTION]**

[Technical Problem]

The present inventors found that in the preparation of a medical film by adding PGA particles to a crosslinked polymer material, non-uniform dispersion of the PGA particles in the medical film fails to enhance the shape retention effect of the medical film. If the retention effect of the film is not enhanced, for example, when used as an adhesion barrier material, the film may tear when a body or an organ moves after the film is attached to the organ, and this may reduce the barrier function for adhesion prevention.

An object of the present invention is to provide a medical film containing particles containing polyglycolic acid and capable of retaining the shape of the medical film in a living body for a long time, and a method for producing the medical film.

[Solution to Problem]

One aspect of the present invention for solving the above problem relates to a medical film of [1] to [5] below.

[1] A medical film containing:

a crosslinked polymer material; and
polyglycolic acid (PGA) dispersoids that are particles containing a polymer having a constituent unit derived from glycolic acid or aggregates of the particles, in which
the polymer having glycolic acid in a constituent unit is a polymer having a ratio of the constituent unit derived from glycolic acid to a total mass of the polymer of 40 mass% or more and 100 mass% or less,
a content of the polyglycolic acid dispersoids per 100 parts by weight of the crosslinked polymer material is 2 parts by weight or more and 40 parts by weight or less, and
a coefficient of variation CV of the number of particles contained in rectangular regions is 0.6 or less, where the coefficient of variation CV is determined from the number of particles measured in each of 16 rectangular regions with a size of 5 mm x 4 mm set so as not to overlap with each other on a surface of the medical film.

[2] The medical film according to [1], in which in the 16 rectangular regions, an average value of the number of aggregates with a diameter that is 5 or more times an average diameter of the polyglycolic acid dispersoids contained in the rectangular regions is 3 or less.

[3] The medical film according to [1] or [2], in which the polymer has a number-average molecular weight of 10000 or more and 150000 or less.

[4] The medical film according to any of [1] to [3], in which the polymer film has a tensile strength of 11 MPa or more and 100 MPa or less.

[5] The medical film according to any of [1] to [4], being an adhesion barrier material.
In addition, one aspect of the present invention for solving the above problem relates to a method for producing a medical film of [6] below.

[6] A method for producing a medical film, the method including:

(a) mixing a solution or dispersion containing a crosslinked polymer material with particles containing a polymer having a constituent unit derived from glycolic acid to form a composite of the crosslinked polymer material and the particles; and
(b) forming the composite of the crosslinked polymer polymer and the particles into a film, in which

the polymer is a polymer having a ratio of the constituent unit derived from glycolic acid to a total mass of the polymer of 40 mass% or more and 100 mass% or less,
a viscosity of the solution or dispersion containing the crosslinked polymer material in step (a) is 2000 mPa·s or more, the viscosity being measured at 25°C and 10 rpm, and
a number-average molecular weight of the polymer in step (a) is 15000 or more and 170000 or less.

[Advantageous Effects of Invention]

According to the present invention, there are provided a medical film containing particles containing polyglycolic acid, capable of retaining the shape of the medical film in a living body for a long time, and less prone to tearing, and a method for producing the medical film.

[Brief Description of Drawings]

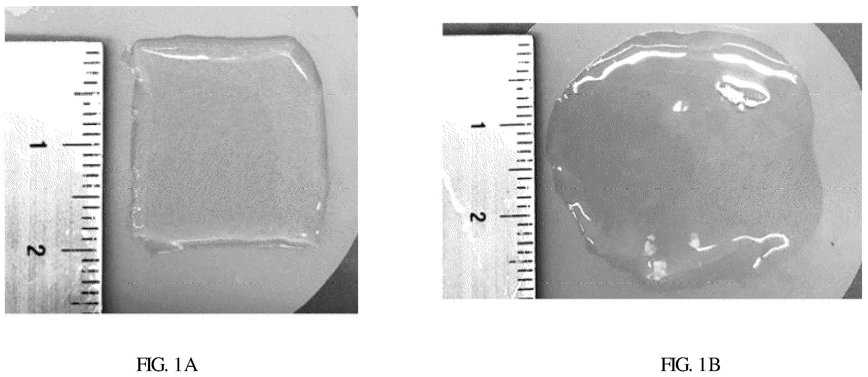

FIG. 1A is a photograph showing an external appearance of a film 4 after immersion in a phosphate buffer solution (PB) of pH 8.0 for 45 hours, and FIG. 1B is a photograph showing an external appearance of a film 9 after immersion in a phosphate buffer solution (PB) of pH 8.0 for 45 hours.

[Description of Embodiments]

Medical Film

One embodiment of the present invention relates to a medical film (hereinafter also referred to simply as the "medical film") containing:

a crosslinked polymer material; and
polyglycolic acid (PGA) dispersoids that are particles containing a polymer having a constituent unit derived from glycolic acid (hereinafter also referred to simply as the "PGA particles") or aggregates of the particles.

Crosslinked Polymer Material

The crosslinked polymer material is a polymer material that swells when immersed in ultrapure water at 15 to 25°C. The crosslinked polymer material is produced by physically crosslinking or chemically crosslinking the same type or different types of polymer materials and is typically a gel polymer. The crosslinked polymer material is preferably composed of a polymer that has solubility after swelling in water.
The crosslinked polymer material can be, for example, a crosslinked product of a polyanionic polymer. The polyanionic polymer may be a polymer having a repeating structure having an anionic group or may be a polymer produced by chemically modifying a polymer compound with an anionic substituent. The anionic group can be a carboxyl group, a hydroxy group, a sulfo group, or the like. The anionic group may partially form a salt with an alkali metal, such as sodium or potassium, or an alkaline earth metal, such as calcium or magnesium.
The polymer material may be a polysaccharide, a protein, or a synthetic polymer. Among them, a polysaccharide or a protein is preferred.

The saccharide as a polyanionic polymer material may be, for example, a natural saccharide, such as pullulan, alginic acid, hyaluronic acid, chondroitin acid, dextran acid, and pectin, or may be a saccharide having an anionic group introduced by modification, such as carboxymethyl amylose, carboxymethyl cellulose, carboxymethyl dextran, carboxymethyl starch, sulfated cellulose, and sulfated dextran. Among them, alginic acid, hyaluronic acid, carboxymethyl amylose, and carboxymethyl cellulose are preferred because they are easy to handle.

Examples of the protein as the polyanionic polymer material include gelatin, collagen, albumin, fibrin, and chemically modified products thereof. The protein may be a polymer of amino acids having an anionic group (polyamino acid), such as polyglutamic acid and polyaspartic acid. Among them, an acid-treated gelatin having a carboxyl group formed by hydrolysis of an acid amide is preferred.

The crosslink of the polymer material may be a physical crosslink formed by introducing a cationic substituent into the polyanionic polymer or may be a chemical crosslink formed by reacting the polymer materials directly or via another molecule. The crosslink of the polymer material can be formed by subjecting the polymer material to a crosslinking treatment, such as ultraviolet light treatment, heat treatment, and crosslinker treatment. Among them, crosslinker treatment is preferred.

Examples of the crosslinker used in the crosslinker treatment include ethyl-3-(3-dimethylaminopropyl)carbodiimide (EDC), N,N'-dicyclohexylcarbodiimide, N,N'-diisopropylcarbodiimide, and N-cyclohexyl-N'-(2-morpholinoethyl) carbodiimide metho-p-toluenesulfonic acid. Treatment with these crosslinkers can introduce a cationic group to the polyanionic polymer. In addition, the molecules of the polymer material can be physically crosslinked by the intermolecular interaction between the anionic group of a certain molecule and the cationic group of another molecule. Among them, EDC is preferred because it is easy to handle.

The crosslinking treatment with a crosslinker can be carried out by dissolving the polymer material in water or an aqueous medium, adding the crosslinker, and stirring the mixture. The state of water or the aqueous medium at this time is adjusted according to the type of crosslinker. For example, when EDC is used as the crosslinker, it is preferable to appropriately add an acid, such as 0.01 M hydrochloric acid, to adjust the pH during the reaction to a low pH (e.g., pH 4.0 to 5.5) and to dissociate the anion group of the polyanionic polymer.

The amount of the crosslinker is preferably 0.5 eq. or more and 10 eq. or less, more preferably 1 eq. or more and 6 eq. or less, and even more preferably 2 eq. or more and 5 eq. or less per reactive functional group of the polymer material (e.g., hyaluronic acid).

The crosslinking may be caused between a plurality of different types of polymers. The crosslinking at this time may be chemical crosslinking caused by reacting these polymers with each other or may be physical crosslinking caused by increasing intermolecular interaction with a crosslinker. For example, from the viewpoint of safety, a crosslinked product of hyaluronic acid and carboxymethyl cellulose in combination can be used as the crosslinked polymer material.

PGA Dispersoids

The PGA dispersoids are particles containing a polymer containing PGA (hereinafter also referred to simply as the "PGA polymer") or aggregates of the particles. In the production of the medical film, the crosslinked polymer material and the PGA particles form a composite and then formed into a film (film formation). At this time, the PGA particles may aggregate to form aggregates. The medical film may contain only PGA particles, may contain only aggregates of the PGA particles, or may contain both of them.

The PGA dispersoids gradually and sustainably release an acid by hydrolysis when the medical film comes into contact with water by attaching the film to an organ or by other means. The sustainably released acid suppresses the disintegration of the crosslinked polymer material and can extend the period during which the film can retain its structure in a living body.

The expression "containing PGA" means that the ratio of the constituent unit derived from glycolic acid to a total mass of the polymer is 40 mass% or more and 100 mass% or less.

In the PGA polymer, the component other than PGA is not particularly limited as long as the ratio of the constituent unit derived from glycolic acid is 40 mass% or more. For example, the polymer can be a homopolymer of glycolic acid or a copolymer of glycolic acid and a hydroxycarboxylic acid, such as lactic acid, hydroxypropionic acid, hydroxybutyric acid, hydroxyvaleric acid, hydroxycaproic acid, or hydroxybenzoic acid, or a lactone, such as caprolactone. In addition, the PGA dispersoids may be a mixture with the above-mentioned polymer and a water-soluble polymer with confirmed biosafety, such as polyethylene glycol, or may be a copolymer of glycolic acid and polyethylene glycol or the like.

The ratio of the PGA polymer contained in the PGA dispersoids is preferably 50 mass% or more and 100 mass% or less, more preferably 60 mass% or more and 100 mass% or less, even more preferably 70 mass% or more and 100 mass% or less, still more preferably 80 mass% or more and 100 mass% or less, and particularly preferably 90 mass% or more and 100 mass% or less relative to a total mass of the PGA dispersoids.

The PGA polymer has a number-average molecular weight of preferably 10000 or more and 150000 or less, more preferably 10000 or more and 100000 or less, and even more preferably 15000 or more and 50000 or less. Reducing the number-average molecular weight increases the sustained-release ability of the acid and can retain the shape of the medical film after being attached to an organ for a longer period of time. In addition, increasing the number-average molecular weight can enhance the dispersibility of the particles.

The number-average molecular weight of the PGA polymer can be determined by measuring the number-average molecular weight of the PGA polymer in the PGA particles separated from the medical film in accordance with ISO 16014-1: 2012. Specifically, gel permeation chromatography (GPC) (Shodex GPC-104 available from Resonac Holdings Corporation; detector: RI; column: HFIP-606M x 2) is used. A solution of $CF_3COONa$ dissolved in 5 mM in hexafluoropropanol (HFIP) is used as a solvent, and poly(methyl methacrylate) (PMMA) is used as a standard material.

The average particle size of the PGA particles is not particularly limited and is preferably 0.1 $\mu$m or more and 30 $\mu$m or less. The PGA particles with a smaller average particle size have larger surface area and a higher degradation rate, and have larger area of contact with the crosslinked polymer material, and can retain the shape of the medical film after attachment to an organ for a longer period. On the other hand, from the viewpoint of suppressing excessive aggregation of the PGA particles and a decrease in dispersion uniformity due to the aggregation, the average particle size of the PGA particles is preferably large to some extent. Thus, the average particle size of the PGA particles is preferably 1 $\mu$m or more and 20 $\mu$m or less, more preferably 1 $\mu$m and 15 $\mu$m or less, and even more preferably 1 $\mu$m or more and 10 $\mu$m or less.

The average particle size of the PGA particles can be measured by observing 200 or more PGA particles that are not aggregated using a digital microscope (available from Keyence Corporation: HVX7000) at a magnification of 100 times to capture digital images. The captured digital image is read into image analysis software, and the lengths of the perimeters of the PGA particles are measured with an image analyzer. The arithmetic mean (average value) and the standard deviation of the lengths of the perimeters of the PGA particles are calculated, measured values with a difference from the average value of three or more times the standard deviation are excluded to determine the average perimeter length, and the equivalent diameter of the particles obtained by dividing the average perimeter length by the ratio (3.1416) of the circumference of a circle to its diameter is defined as the average particle size of the PGA particles.

The image processing is performed by reading the digital image into MIPAR (available from Lightstone Corp.) and setting the conditions of the image analysis software as follows.

"Smart Cluster" is set to "Fill Type: Class", "Classes: 4 to 10", "Edge Type: Dark to Bright", "Edge Clean: 0 to 5", and "Speed: 2".
"Basic Threshold" is set to "Value: 80 to 150".
"Reject Features" is set to "Measurement: Area", "Target: Objects", "Edges: Include", "Units: $\mu$m$^2$", "Threshold Value: 100", and "Type: Reject Features </=".

Increasing the mixing ratio of the PGA dispersoids to the crosslinked polymer material can extend the shape retention duration of the medical film is retained in a living body. On the other hand, adjusting the mixing ratio to an appropriate range can suppress excessive aggregation of the PGA particles, increase the dispersion uniformity, and suppress resulting shortening of the shape retention period of the medical film. The mass ratio of the PGA dispersoids to 100 parts by mass of the crosslinked polymer material is 2 parts by mass or more and 40 parts by mass or less, preferably 8 parts by mass or more and 30 parts by mass or less, more preferably 10 parts by mass or more and 30 parts by mass or less, even more preferably 15 parts by mass or more and 30 parts by mass or less, and particularly preferably 20 parts by mass or more and 30 parts by mass or less.

The mass ratio of the PGA dispersoids contained in the medical film can be determined by removing the crosslinked polymer material, measuring the mass of the remaining particles as the mass of the PGA dispersoids and measuring the mass of the removed crosslinked polymer material, and calculating the mass of the PGA dispersoids relative to the mass of the crosslinked polymer material. For the crosslinked polymer material that can be dissolved and removed, such as hyaluronic acid and its crosslinked product, the material can be dissolved and removed using an appropriate acid compound. Then, a value obtained by dividing the mass of water contained in the film and the mass of the residue, such as the PGA dispersoids remaining after the removal, from the mass of the medical film before the removal can be defined as the mass of the crosslinked polymer material.

In the medical film according to the present embodiment, excessive aggregation of the PGA particles is suppressed, and this results in uniform dispersion of the PGA particles. This can increase the degradation rate of the PGA particles and increase the area of contact with the crosslinked polymer material, thereby extending the period during which the shape of the medical film can be retained.

In the present embodiment, a coefficient of variation is used as an index representing the dispersibility of the PGA particles in the medical film. The coefficient of variation is not affected by the average particle size of the PGA particles and thus can evaluate the dispersibility of particles with different average particle sizes.

Specifically, the medical film has a coefficient of variation CV of the number of particles contained in rectangular regions of 0.6 or less, where the coefficient of variation CV is determined from the number of PGA particles measured in each of 16 rectangular regions with a size of 5 mm x 4 mm set so as not to overlap with each other on a surface of the medical film. The coefficient of variation CV is an index indicating the degree of uniform dispersion of the PGA particles in the medical film. A smaller coefficient of variation CV indicates that the PGA particles are more uniformly dispersed in the medical film. The coefficient of variation CV is preferably 0.5 or less, more preferably 0.4 or less, even more preferably 0.3 or less, and particularly preferably 0.2 or less.

The coefficient of variation CV can be calculated by the following method.

On the surface of the medical film, 16 rectangular regions selected by the following method are set and observed at a magnification of 100 times using a digital microscope (available from Keyence Corporation: HVX7000) to capture digital images. The captured images are read into image analysis software, and the number of PGA particles contained in each rectangular region is determined from the lengths of the perimeters of the PGA dispersoids. Specifically, the average number of particles and the standard deviation of number of particles of the PGA particles in the 16 rectangular regions are calculated by the following method, and the calculated standard deviation is divided by the average number of particles to determine the coefficient of variation CV of the number of particles contained in the rectangular regions. The image analysis can be performed under the same conditions as in the calculation of the average particle size of the PGA particles described above.

At this time, one side of the medical film is defined as the x-axis, the other side of the film in the direction perpendicular to the x-axis is defined as the y-axis, and the intersection of the x-axis and the y-axis is defined as a coordinate point x0y0. The medical film is divided into five equal parts in the x-axis direction, and coordinate points x0y0, x1y0, x2y0, ..., x5y0 are set from x0y0 along the x-axis. Similarly, the medical film is divided into five equal parts in the y-axis direction, and coordinate points x0y0, x0y1, x0y2, ..., x0y5 are set from x0y0 along the y-axis. 16 rectangular regions each having a size of 5 mm x 4 mm and containing 16 points of x1y1, x2y1, x3y1, x4y1, x1y2, x2y2, x3y2, x4y2, x1y3, x2y3, x3y3, x4y3, x1y4, x2y4, x3y4, and x4y4 in this coordinate plane are set so as not to overlap with each other, and each rectangular region is defined as a rectangular region i (i is an integer of 1 to 16). In addition, also when the plan view of the medical film is not substantially rectangular, 16 rectangular regions are equally defined from the entire planar direction of the medical film in the plan view according to the same concept.

Then, the length of the perimeter of a PGA dispersoid j observed in the rectangular region i is defined as $L_{(j)}$. The PGA dispersoid and PGA particle are assumed to be spherical, and the diameter of the dispersoid is assumed to be proportional to the length of the perimeter of the PGA dispersoid, and the diameter of the PGA particle is assumed to be proportional to the length of the perimeter of the PGA particle. Based on this assumption, the number $n_{(j)}$ of particles constituting the PGA dispersoid j is determined by Equation 1 from a length $L_0$ of the perimeter of one PGA particle and the length $L_{(j)}$ of the perimeter of the PGA dispersoid j. In the following calculation, the length $L_0$ of the perimeter of the PGA particle determined by tentatively setting the average particle size or the lengths of the perimeters of the PGA particles is used. For example, in the following calculation, the average particle size of the PGA particles can be assumed to be 10 $\mu$m.

[Math. 1]

## Equation 1

$$n_{(j)} = \frac{L_{(j)}^{3}}{L_0^{3}}$$

From the number $n_{(j)}$ of the PGA particles contained in each of N PGA dispersoids observed in the rectangular region i, the number X(i) of the PGA particles contained in the rectangular region i is determined by Equation 2.

[Math. 2]

## Equation 2

$$X_{(i)} = \sum_{j=1}^{N} n_{(j)} = \sum_{j=1}^{N} \frac{L_{(j)}^{3}}{L_0^{3}}$$

An average number $X_{(ave)}$ of particles, which is the average value of the numbers of particles contained in each of the 16 rectangular regions, is determined by Equation 3.

[Math. 3]

Equation 3

$$X_{(ave)} = \frac{1}{16} \sum_{i=1}^{16} X_{(i)} = \frac{1}{16} \sum_{i=1}^{16} \left( \sum_{j=1}^{N} \frac{L_{(j)}^3}{L_0^3} \right)$$

Then, a standard deviation S of the number of particles observed in each rectangular region is determined, and the standard deviation S is divided by the average number $X_{(ave)}$ of particles to determine the coefficient of variation CV of the number of particles contained in the rectangular regions by Equation 4.

[Math. 4]

Equation 4

$$CV = \frac{S}{X_{(ave)}} = \frac{\sqrt{\frac{1}{16} \sum_{i=1}^{16} \left( X_{(i)} - X_{(ave)} \right)^2}}{X_{(ave)}}$$

The dispersibility of the PGA particles can also be evaluated by the average diameter of the PGA dispersoids in the medical film or the average value of the number of aggregates (hereinafter also referred to simply as the "5A aggregates") with a diameter that is 5 or more times the average diameter of the PGA dispersoids in the rectangular region set as described above. Specifically, in the medical film, the average diameter of the PGA dispersoids is preferably 10 $\mu$m or more and 60 $\mu$m or less, more preferably 25 $\mu$m or more and 50 $\mu$m or less, and even more preferably 30 $\mu$m or more and 45 $\mu$m or less. In addition, in the medical film, the average value of the number of the 5A aggregates in the rectangular regions is preferably 3 or less, more preferably 2 or less, even more preferably 1 or less, and particularly preferably 0.

The average diameter of the PGA dispersoids and the average value of the number of the 5A aggregates in the rectangular regions can be calculated by the following methods.

Each rectangular region is observed at a magnification of 100 times using a digital microscope to capture a digital image. The captured digital image is read into image analysis software, and the lengths of the perimeters of the PGA dispersoids contained in each rectangular region are determined by the same method as that for calculation of the coefficient of variation CV. On the assumption that the PGA dispersoids are spheres, a diameter $D_{(j)}$ of the j-th dispersoid i of N dispersoids observed in the rectangular region i is determined by Equation 5 by dividing the length $L_{(j)}$ of the perimeter of the PGA dispersoid determined by image processing by the ratio $\pi$ (3.1416) of the circumference of a circle to its diameter.

[Math. 5]

Equation 5

$$D_{(j)} = \frac{L_{(j)}}{\pi}$$

Then, an average diameter $D_{(i)}$ of the dispersoids contained in the rectangular region i is determined by Equation 6.

[Math. 6]

Equation 6

$$D_{(i)} = \frac{1}{N} \sum_{j=1}^{N} D_{(j)} = \frac{1}{N \times \pi} \sum_{j=1}^{N} L_{(j)}$$

Furthermore, an average value $D_{(ave)}$ of the average diameters $D_{(i)}$ of the dispersoids in the 16 rectangular regions is determined by Equation 7. In the present embodiment, this $D_{(ave)}$ is defined as the average diameter of the PGA dispersoids.

[Math. 7]

## Equation 7

$$D_{(ave)} = \frac{1}{16} \sum_{i=1}^{16} D_{(i)}$$

In the same manner, in each of the 16 rectangular regions, an average diameter $D_{(i)}$ of the aggregates and the number $A_{(i)}$ of the aggregates with a diameter of 5 or more times $D_{(i)}$ are measured. An average value $A_{(ave)}$ of the number $A_{(i)}$ of the aggregates with a diameter of 5 or more times $D_{(i)}$ in the 16 rectangular regions is determined by Equation 8. In the present embodiment, this $A_{(ave)}$ is defined as the average value of the number of the 5A aggregates in the rectangular regions.
[Math. 8]

## Equation 8

$$A_{(ave)} = \frac{1}{16} \sum_{i=1}^{16} A_{(i)}$$

Additional Component

The medical film may contain a lubricant, an emulsifier, a colorant, an antioxidant, a weather-resistant agent, a heat stabilizer, a crystal nucleating agent, an ultraviolet absorber, a colorant, an antibacterial agent, and/or the like. The content of these components is preferably 0 mass% or more and 20 mass% or less, more preferably 0 mass% or more and 10 mass% or less, and even more preferably 0 mass% or more and 5 mass% or less relative to the total mass of the medical film.

Strength

Uniform dispersion of the PGA particles can increase the strength of the medical film when it is wet with water, and this can make the medical film less prone to tearing during the operations of attaching and reattaching. The tensile strength of the medical film in a wet state with water is preferably 11 MPa or more, more preferably 13 MPa or more, and even more preferably 15 MPa or more. The upper limit value of the tensile strength is not particularly limited and can be 100 MPa or less.

Method for Producing Medical Film

The medical film can be produced by crosslinking a polymer material, forming a composite of the crosslinked polymer material and the PGA particles, and forming the composite into a film (film formation).

The crosslinking of the polymer material is performed by a method suitable for the polymer, such as ultraviolet treatment, heat treatment, and crosslinker treatment. For example, crosslinking with EDC as a crosslinker can be performed by adding EDC to a solution of the polymer dissolved in water or an aqueous medium with stirring. At this time, an acid, such as HCl, is preferably added to adjust the solution to acidic conditions (e.g., pH 4.0 to 5.5). After the crosslinking treatment with a crosslinker, an unnecessary substance is preferably removed by dialysis or the like.

The composite with the PGA particles is formed by adding the PGA particles to a solution or dispersion containing the crosslinked polymer material and stirring and mixing.

The film is formed by drying the composite of the crosslinked polymer material and the PGA particles in a frame. The drying may be performed by air drying at ordinary temperature, freeze drying, or removing a solvent by heating. In addition, hot pressing (heating and pressurization) may be performed. The drying may be performed by combining a plurality of methods.

On the other hand, according to the findings of the present inventors, reducing the molecular weight of the PGA polymer, reducing the particle sizes of the PGA particles, or the like to enhance the shape retention effect would promote aggregation of the PGA particles and non-uniform dispersion of the PGA particles. Excessive aggregation of the PGA particles and the resulting non-uniform dispersion would make it difficult to exhibit the shape retention effect of the film by the PGA particles. In addition, excessive aggregation of the PGA particles and the resulting non-uniform dispersion would reduce the tensile strength of the medical film when the film is wet with water and make the film prone to tearing. Thus, this would make it difficult to attach the medical film as an adhesion barrier material to an organ or to reattach the medical film to an organ after being peeled off from another organ to which the film is once attached. In addition, the medical film attached to an organ as an adhesion barrier material may attach to another organ besides the target organ when the organ to which the film is attached or a human body moves. Thus, to extend the shape retention time of the medical film during which the shape can be retained in a living body and to make the medical film less prone to tearing also when the medical film is wet with water, it is important to uniformly disperse the PGA particles and suppress excessive aggregation.

In addition, in the formation of the composite, the dispersibility of the PGA particles can be adjusted to suppress excessive aggregation by adjusting the viscosity of the solution or dispersion containing the crosslinked polymer material, the average particle size of the PGA particles, and/or the amount of the PGA particles to be added. From the above viewpoint, the viscosity of the solution or dispersion containing the crosslinked polymer material measured at 25°C and 10 rpm is preferably 2000 mPa·s or more and 60000 mPa·s or less, more preferably 3000 mPa·s or more and 60000 mPa·s or less, and even more preferably 5000 mPa·s or more and 50000 mPa·s or less. Furthermore, from the viewpoint of increasing the uniformity of thickness in forming the film, the viscosity of the solution or dispersion containing the crosslinked polymer material measured at 25°C and 1 rpm is preferably 5000 mPa·s or more, more preferably 8000 mPa·s or more and 300000 mPa·s or less, and even more preferably 15000 mPa·s or more and 150000 mPa·s or less.

The particle sizes of the PGA particles for forming the composite can be prepared by the number-average molecular weight of the PGA polymer constituting the PGA particles. The particles with higher number-average molecular weight tend to be harder, and when milled, the particles tend to be larger. The number-average molecular weight of the PGA polymer is preferably 15000 or more and 170000 or less, more preferably 20000 or more and 170000 or less, and even more preferably 30000 or more and 170000 or less. The number-average molecular weight of the PGA polymer may decrease during the production of the film.

Usually, to increase the dispersibility of particulate matter added to sol, the viscosity of the sol is reduced. However, reducing the concentration by adding a solvent to reduce the sol viscosity would lead to movement of the dispersed PGA particles in the sol with a low viscosity during drying after casting, and this would promote aggregation and/or deviation in distribution of the PGA particles. In addition, to produce a film with a certain thickness or more when the concentration is reduced, the casting amount needs to be increased. Increasing the casting amount would result in uneven distribution of the sol during drying after casting and be likely to cause thickness deviation. Furthermore, increasing the casting amount would promote further aggregation and/or deviation in distribution of the particles due to the movement of the particles during drying. In contrast, in the present embodiment, increasing the viscosity of the sol prevents excessive aggregation of the particles and also makes it less likely to cause the thickness deviation of the film.

Moreover, increasing the viscosity of the solution or dispersion containing the crosslinked polymer material can make the solution or dispersion less likely to spill from a casting frame when the solution or dispersion is placed in a dryer after casting, facilitating the production of the medical film. On the other hand, reducing the viscosity to some extent can facilitate the dispersion of the PGA particles and liquid feeding during production. Thus, a medical film with high dispersibility of the PGA particles can be produced by combining a sol with a viscosity as described above and the PGA particles.

Adjusting these conditions can increase the dispersibility of the PGA particles and can reduce the amount of coarse aggregates in which the PGA particles aggregate in the medical film. In addition, this can suppress the shortening of the period during which the structure can be retained in a living body, the decrease in strength in a living body, and the like due to the aggregation of the PGA particles.

The film thus produced can be used as a medical film.

Applications

The medical film can be attached to an organ or the like, starts to change in shape (disintegrate) by degradation after a predetermined time has elapsed, and is finally absorbed into a living body. The period from the attachment to the occurrence of the shape change can be adjusted to a period of 3 days to 14 days. In addition, the period from the attachment to the time when the medical film is absorbed into a living body and becomes invisible can be adjusted to a period of 14 days to 28 days. These periods can be adjusted according to the type of crosslinked polymer material and the type of biodegradable resin.

The characteristics described above enable the medical film to be used as a covering material for an affected area for covering a wound site caused by surgery to prevent adhesion (adhesion barrier material), for preventing the occurrence of an ulcer or a perforation, and for other purposes. The medical film can also be used as a substrate for sustained release of a drug or a substrate for tissue generation. Alternatively, the medical film can also be used as a cavity holding material for holding a cavity by being applied to a wall surface of a lumen in a living body.

When used for various applications, the medical film may be used alone or used as a laminate with another layer.

Other Embodiments

The embodiments described above are exemplary embodiments of the present invention, and needless to say, the present invention can include embodiments other than the embodiments described above within the scope of the technical idea constituting the core of the present invention.

[Examples]

The present invention will be described in detail below based on examples, but the present invention is not limited to these examples.

1. Production of Medical Film

1-1. Production of Crosslinked Polymer Material

1-1-1. Production of HA/CMC Condensate-1

Hyaluronic acid (HA) (available from Kewpie Corporation, molecular weight 2200000 to 2500000) in an amount of 1650 mg and 750 mg of carboxymethyl cellulose (CMC) (available from Tokyo Chemical Industry Co., Ltd., molecular weight 250000, degree of etherification from 0.5 to 0.8) were weighed. HA and CMC were added to a beaker containing 300 mL of ultrapure water and stirred until completely dissolved. Then, 0.1 N HCl was added to adjust the pH of the content to 4.80, and an HA/CMC solution was prepared. Ethyl-3-(3-dimethylaminopropyl)carbodimide (EDC) (available from Dojindo Laboratories) in an amount of 3180 mg was weighed and dissolved in 7.5 mL of ultrapure water, and an EDC solution was prepared. While the HA/CMC solution was stirred at a stirring speed of 600 rpm and an appropriate amount of 0.1 N HCl was added to prepare the pH to 4.70 to 5.10, the entire volume of the EDC solution was added. The mixture was stirred for about 120 minutes until the pH did not change, and a reaction solution was obtained. Finally, the reaction solution after stirring was placed in a dialysis membrane with a molecular weight cut-off of 12000 to 14000 and stirred in ultrapure water. The entire volume of the dialysate (ultrapure water) was exchanged three times during 36 hours from the start of dialysis, and an HA/CMC condensate-1 (a solution of a crosslinked polymer material) was obtained.
The concentration of the HA/CMC condensate-1 ((mass of HA and mass of CMC used as materials)/(mass of HA, CMC, and ultrapure water used as materials)) was 0.77 wt.%, the solution viscosity measured at 25°C and 1 rpm was about 30000 mPa·s, and the viscosity measured at 10 rpm was about 8000 mPa·s.
The HA/CMC condensate-1 was diluted with ultrapure water, and an HA/CMC condensate-2 (a solution of a crosslinked polymer material) was obtained. The concentration of the HA/CMC condensate-2 ((mass of HA and mass of CMC used as materials)/(mass of HA, CMC, ultrapure water used as materials, and ultrapure water for dilution)) was 0.67 wt.%, the solution viscosity measured at 25°C and 1 rpm was about 11000 mPa·s, and the viscosity measured at 10 rpm was about 4000 mPa·s.
The HA/CMC condensate-1 was diluted with ultrapure water, and an HA/CMC condensate-3 (a solution of a crosslinked polymer) was obtained. The concentration of the HA/CMC condensate-3 ((mass of HA and mass of CMC used as materials)/(mass of HA, CMC, ultrapure water used as materials, and ultrapure water for dilution)) was 0.41 wt.%, the solution viscosity measured at 25°C and 1 rpm was about 1500 mPa·s, and the viscosity measured at 10 rpm was about 950 mPa·s.

1-2. Production of Medical Film

Particles of biodegradable polyesters shown in Table 1 were prepared. These particles were all passed through a sieve with an opening of 38 μm and used.

[Table 1]

| Particle composition | Resin name | Raw material composition (mass%) | | Number-average molecular weight |
| --- | --- | --- | --- | --- |
| | | Glycolic acid | Lactic acid | |
| Particles 1 | Polyglycolic acid (PGA) powder | 100 | 0 | 5000 |
| Particles 2 | Polyglycolic acid (PGA) powder | 100 | 0 | 11000 |
| Particles 3 | Polyglycolic acid (PGA) powder | 100 | 0 | 30000 |
| Particles 4 | Polyglycolic acid (PGA) powder | 100 | 0 | 164000 |
| Particles 5 | Polyglycolic acid-polylactic acid copolymer (PGLA) powder | 21 | 79 | from 4000 to 15000 |

(continued)

| Particle composition | Resin name | Raw material composition (mass%) | | Number-average molecular weight |
|---|---|---|---|---|
| | | Glycolic acid | Lactic acid | |
| Particles 6 | Polyglycolic acid-polylactic acid copolymer powder | 45 | 55 | from 7000 to 17000 |
| Particles 7 | Polyglycolic acid-polylactic acid copolymer (PGLA) powder | 21 | 79 | 30000 |
| Particles 8 | Polyglycolic acid-polylactic acid copolymer (PGLA) powder | 45 | 55 | 30000 |
| Particles 9 | Polylactic acid polymer (PLA) powder | 0 | 100 | from 10000 to 18000 |
| Particles 10 | Polylactic acid polymer (PLA) powder | 0 | 100 | 100000 |

1-2-1. Production of Film 1

The HA/CMC condensate-1 in an amount of 29.8 g and 62.7 mg of biodegradable polyester particles (particles 3) were weighed and added to a beaker. The content of the beaker to which the particles 3 were added was sonicated for 1 minute with stirring with a spatula. A 6 x 6-cm frame (frame thickness of 2 cm) was prepared on a glass plate, and the HA/CMC condensate-1 to which the particles 3 were added was poured into the frame. At a temperature of 30°C, the glass plate was placed on a rotating sample table and dried for about 3 days while the glass plate was rotated. After drying, a film was peeled off from the glass plate and heat-treated using a hot press machine at 90°C for 15 minutes and then at 120°C for 10 minutes, and a film 1 was obtained.

1-2-2. Production of Film 2

A film 2 was obtained in the same manner as in the production of the film 1 except for changing the biodegradable polyester particles to particles 4 and adding 10.5 mg of the particles 4.

1-2-3. Production of Film 3

A film 3 was obtained in the same manner as in the production of the film 1 except for changing the biodegradable polyester particles to the particles 4 and adding 20.9 mg of the particles 4.

1-2-4. Production of Film 4

A film 4 was obtained in the same manner as in the production of the film 1 except for changing the biodegradable polyester particles to the particles 4 and adding 62.7 mg of the particles 4.

1-2-5. Production of Film 5

A film 5 was obtained in the same manner as in the production of the film 1 except for adding 59.6 mg of the HA/CMC condensate-1 and adding 62.7 mg of the particles 4.

1-2-6. Production of Film 6

A film 6 was obtained in the same manner as in the production of the film 1 except for changing the biodegradable polyester particles to particles 6 and adding 62.7 mg of particles 6.

1-2-7. Production of Film 7

A film 7 was obtained in the same manner as in the production of the film 1 except for changing the HA/CMC condensate-1 to the HA/CMC condensate-2, adding 33.8 g of the HA/CMC condensate-2, and adding 10.5 mg of the particles 4.

1-2-8. Production of Film 8

A film 8 was obtained in the same manner as in the production of the film 1 except for changing the biodegradable polyester particles to particles 8.

1-2-9. Production of Film 9

A film 9 was obtained in the same manner as in the production of the film 1 except for changing the biodegradable polyester particles to particles 1.

1-2-10. Production of Film 10

A film 10 was obtained in the same manner as in the production of the film 1 except for adding 59.6 g of the HA/CMC condensate-1, changing the biodegradable polyester particles to the particles 1, and adding 67.7 mg of the particles 1.

1-2-11. Production of Film 11

A film 11 was obtained in the same manner as in the production of the film 1 except for changing the biodegradable polyester particles to particles 2.

1-2-12. Production of Film 12

A film 12 was obtained in the same manner as in the production of the film 1 except for changing the biodegradable polyester particles to particles 5.

1-2-13. Production of Film 13

A film 13 was obtained in the same manner as in the production of the film 1 except for changing the biodegradable polyester particles to particles 9.

1-2-14. Production of Film 14

A film 14 was obtained in the same manner as in the production of the film 1 except for changing the biodegradable polyester particles to particles 10.

1-2-15. Production of Film 15

A film 15 was obtained in the same manner as in the production of the film 1 except for changing the biodegradable polyester particles to the particles 4 and adding 2.1 mg of the particles 4.

1-2-16. Production of Film 16

A film 16 was obtained in the same manner as in the production of the film 1 except for changing the HA/CMC condensate-1 to the HA/CMC condensate-3, adding 55.8 g of the HA/CMC condensate-3, changing the biodegradable polyester particles to the particles 4, and adding 10.5 mg of the particles 4.

1-2-17. Production of Film 17

A film 17 was obtained in the same manner as in the production of the film 1 except for changing the biodegradable polyester particles to particles 7 and adding 62.7 mg of particles 7.

Table 2 shows the compositions of the HA/CMC condensates, the viscosities of the solutions, and the added amounts of the HA/CMC condensates used in the production of each sample; the compositions, particle sizes, and added amounts of the PGA particles used in the production of each sample; and the parts by weight of the particles added per 100 parts by weight of the HA/CMC.

[Table 2]

| | Sample | HA/CMC condensate | | | Particles | | | Parts by weight of particles per 100 parts by weight of HA/CMC |
|---|---|---|---|---|---|---|---|---|
| | | HA/CMC concentration / wt.% | Solution viscosity / mPa·s | Added amount / g | Particle composition | Particle size / μm | Added amount / mg | |
| Example 1 | Film 1 | 0.77 | 8000 | 29.8 | Particles 3 | 6.86 | 62.7 | 27.3 |
| Example 2 | Film 2 | 0.77 | 8000 | 29.8 | Particles 4 | 9.85 | 10.5 | 4.6 |
| Example 3 | Film 3 | 0.77 | 8000 | 29.8 | Particles 4 | 9.85 | 20.9 | 9.1 |
| Example 4 | Film 4 | 0.77 | 8000 | 29.8 | Particles 4 | 9.85 | 62.7 | 27.3 |
| Example 5 | Film 5 | 0.77 | 8000 | 59.6 | Particles 4 | 9.85 | 62.7 | 13.7 |
| Example 6 | Film 6 | 0.77 | 8000 | 29.8 | Particles 6 | 7.48 | 62.7 | 27.3 |
| Example 7 | Film 7 | 0.67 | 4000 | 33.8 | Particles 4 | 9.85 | 10.5 | 4.6 |
| Example 8 | Film 8 | 0.77 | 8000 | 29.8 | Particles 8 | 7.89 | 62.7 | 27.3 |
| Comparative Example 1 | Film 9 | 0.77 | 8000 | 29.8 | Particles 1 | 2.56 | 62.7 | 27.3 |
| Comparative Example 2 | Film 10 | 0.77 | 8000 | 59.6 | Particles 1 | 2.56 | 67.7 | 14.8 |
| Comparative Example 3 | Film 11 | 0.77 | 8000 | 29.8 | Particles 2 | 4.62 | 62.7 | 27.3 |
| Comparative Example 4 | Film 12 | 0.77 | 8000 | 29.8 | Particles 5 | 6.32 | 62.7 | 27.3 |
| Comparative Example 5 | Film 13 | 0.77 | 8000 | 29.8 | Particles 9 | 5.31 | 62.7 | 27.3 |
| Comparative Example 6 | Film 14 | 0.77 | 8000 | 29.8 | Particles 10 | 6.79 | 62.7 | 27.3 |
| Comparative Example 7 | Film 15 | 0.77 | 8000 | 29.8 | Particles 4 | 9.85 | 2.1 | 0.9 |
| Comparative Example 8 | Film 16 | 0.41 | 950 | 55.8 | Particles 4 | 9.85 | 10.5 | 4.6 |
| Comparative Example 9 | Film 17 | 0.77 | 8000 | 29.8 | Particles 7 | 7.32 | 62.7 | 27.3 |

2. Evaluation

2-1. Calculation of Coefficient of Variation of Number of Particles

On each surface of films 1 to 17, 16 rectangular regions selected by the following method were set and observed at a magnification of 100 times using a digital microscope (available from Keyence Corporation: HVX7000), and digital images were captured. The captured images were read into image analysis software (available from Lightstone Corp.: MIPAR), and the number of the biodegradable polyester particles contained in each rectangular region was determined from the lengths of the perimeters of the particles or their aggregates (dispersoids). The average number of particles and the standard deviation of number of particles in the rectangular regions of the 16 rectangular regions were calculated by the following method, the standard deviation was divided by the average number of particles, and the coefficient of variation CV of the number of particles contained in the rectangular regions was determined.

In identifying the dispersoids and measuring the lengths of the perimeters, each condition of the image analysis software was set as follows.

"Smart Cluster" was set to "Fill Type: Class", "Classes: 4 to 10", "Edge Type: Dark to Bright", "Edge Clean: 0 to 5", and "Speed: 2".

"Basic Threshold" was set to "Value: 80 to 150".

At this time, one side of the medical film was defined as the x-axis, the other side of the film in the direction perpendicular to the x-axis was defined as the y-axis, and the intersection of the x-axis and the y-axis was expressed as a coordinate point x0y0. The film was divided into five equal parts in the x-axis direction, and coordinate points x0y0, x1y0, x2y0, ..., x5y0 were set from x0y0 along the x-axis. Similarly, the film was divided into five equal parts in the y-axis direction, and coordinate points x0y0, x0y1, x0y2, ..., x0y5 were set from x0y0 along the y-axis. Rectangular regions each having a size of 5 mm x 4 mm and containing 16 points of x1y1, x2y1, x3y1, x4y1, x1y2, x2y2, x3y2, x4y2, x1y3, x2y3, x3y3, x4y3, x1y4, x2y4, x3y4, and x4y4 in this coordinate plane were set so as not to overlap with each other and were each defined as a rectangular region i (i is an integer of 1 to 16). The length of the perimeter of the dispersoid j observed in the rectangular region i was defined as $L_{(j)}$. The dispersoid and particle were assumed to be spherical, and the diameter of the dispersoid was assumed to be proportional to the length of the perimeter of the dispersoid, and the diameter of the particle was assumed to be proportional to the length of the perimeter of the particle. Based on this assumption, the number $n_{(j)}$ of particles constituting the dispersoid j was determined by Equation 1 from a length L0 of the perimeter of one particle and the length $L_{(j)}$ of the perimeter of the dispersoid j. The length L0 of the perimeter of the particle was determined on the assumption that the average particle size of the particles was 10 $\mu$m.

[Math. 9]

## Equation 1

$$n_{(j)} = \frac{L_{(j)}^{3}}{L_0^{3}}$$

From the number $n_{(j)}$ of particles contained in each of N dispersoids observed in the rectangular region i, the number X(i) of particles contained in the rectangular region i was determined by Equation 2.

[Math. 10]

## Equation 2

$$X_{(i)} = \sum_{j=1}^{N} n_{(j)} = \sum_{j=1}^{N} \frac{L_{(j)}^{3}}{L_0^{3}}$$

An average number $X_{(ave)}$ of particles, which was the average value of the numbers of particles contained in each of the 16 rectangular regions, was determined by Equation 3.

[Math. 11]

## Equation 3

$$X_{(ave)} = \frac{1}{16} \sum_{i=1}^{16} X_{(i)} = \frac{1}{16} \sum_{i=1}^{16} \left( \sum_{j=1}^{N} \frac{L_{(j)}^{3}}{L_0^{3}} \right)$$

A standard deviation S of the number of particles observed in each rectangular region was determined, the standard deviation S was divided by the average number $X_{(ave)}$ of particles, and the coefficient of variation CV of the number of particles contained in the rectangular regions was determined by Equation 4.

[Math. 12]

## Equation 4

$$CV = \frac{S}{X_{(ave)}} = \frac{\sqrt{\frac{1}{16} \Sigma_{i=1}^{16} \left( X_{(i)} - X_{(ave)} \right)^2}}{X_{(ave)}}$$

2-2. Average Diameter of Aggregates

Each rectangular region was observed using a digital microscope (available from Keyence Corporation: HVX7000) at a magnification of 100 times, and a digital image was obtained. The resulting digital image was read into image analysis software, and the lengths of the perimeters of the dispersoids contained in each rectangular region were determined by the same method as that for calculation of the coefficient of variation CV. On the assumption that the dispersoids were spheres, the diameter $D_{(j)}$ of the j-th dispersoid i of N dispersoids observed in the rectangular region i was determined by Equation 5 by dividing the length $L_{(j)}$ of the perimeter of the dispersoid determined by image processing by the ratio $\pi$ (3.1416) of the circumference of a circle to its diameter.

[Math. 13]

# Equation 5

$$D_{(j)} = \frac{L_{(j)}}{\pi}$$

Then, an average diameter $D_{(i)}$ of the dispersoids contained in the rectangular region i was determined by Equation 6. Furthermore, an average value $D_{(ave)}$ of the average diameters $D_{(i)}$ of the dispersoids in the 16 rectangular regions was determined by Equation 7.

[Math. 14]

# Equation 6

$$D_{(i)} = \frac{1}{N}\sum_{j=1}^{N} D_{(j)} = \frac{1}{N \times \pi}\sum_{j=1}^{N} L_{(j)}$$

[Math. 15]

# Equation 7

$$D_{(ave)} = \frac{1}{16}\sum_{i=1}^{16} D_{(i)}$$

2-3. Average Number of 5A Particles in Rectangular Region

In each of the 16 rectangular regions, an average diameter $D_{(i)}$ of the aggregates and the number $A_{(i)}$ of the aggregates with a diameter of 5 times or more of $D_{(i)}$ were measured. An average value $A_{(ave)}$ of the number $A_{(i)}$ of the aggregates with a diameter of 5 times or more of $D_{(i)}$ in the 16 rectangular regions was determined by Equation 8.

[Math. 16]

# Equation 8

$$A_{(ave)} = \frac{1}{16}\sum_{i=1}^{16} A_{(i)}$$

2-4. Degradability

In 8 mL of a phosphate buffer solution (pH 8.0), a 1-$cm^2$ sample (dimension: 1 cm x 1 cm) of each material of films 1 to 14 was immersed and stored at 37°C. The external appearance of the shape of the sample was observed after 24 h, 40 h, and 45 h elapsed from the immersion, and the shape was evaluated. A sample with a score of 2 points or more according to the rating below was determined to retain a film-like shape.

For the results of the external appearance observation, the following scores were set from the image of the external appearance of each sample, and the results were expressed by average values of the scores determined by a plurality (5) of experienced and skilled technicians with reference to the images.

4 Points: the external appearance is substantially the same as the planar shape of the sample before the immersion

3 Points: in the external appearance, the sample is entirely expanded from the planar shape before the immersion, but the presence of the sample can be visually confirmed

2 Points: in the external appearance, the sample is entirely expanded from the planar shape before the immersion and includes slightly disintegrated part, but the presence of the sample can be visually confirmed

1 Point: in the external appearance, the sample is entirely disintegrated or confirmed to be an indeterminate form with a droplet

0 Points: the sample is dissolved, and the external appearance cannot be confirmed

2-5. Strength

The films 1 to 14, 17, and 18 were cut into strips with a long side of 40 mm and a short side of 10 mm, and these were used as samples. The tensile strengths of the samples wetted with ultrapure water were measured using a creep meter (RE2-33005B available from Yamaden Co., Ltd.). The sample was fixed to chucks so that the distance between the chucks was 20 mm. Berklin (product No. E-2 available from AION Co., Ltd.) soaked with ultrapure water was brought into close contact with a central portion of 10 mm x 10 mm of one surface of the sample, and the sample was allowed to stand for 10 seconds. After 10 seconds, the sample was pulled at 0.1 mm/sec to measure the tensile strength (MPa).

The results of Evaluations 2-1 to 2-5 of the films 1 to 17 are shown in Table 3.

FIG. 1A is a photograph showing an external appearance of the film 4 after immersion for 45 hours, and FIG. 1B is a photograph showing an external appearance of the film 9 after immersion for 45 hours.

[Table 3]

| | | Coefficient of variation CV | Average diameter $D_{(ave)}$ /$\mu$m | Average number of 5A particles $A_{(ave)}$ | Degradability | | | Tensile strength after contact with water for 10 sec / MPa |
|---|---|---|---|---|---|---|---|---|
| | | | | | 24 h | 40 h | 45 h | |
| | Example 1 | 0.41 | 31.7 | 2 | 4 | 2 | 2 | 16.3 |
| | Example 2 | 0.14 | 34.4 | 1 | 4 | 2 | 2 | 19.9 |
| | Example 3 | 0.23 | 27.1 | 0 | 4 | 2 | 2 | 13.6 |
| | Example 4 | 0.39 | 32.6 | 0 | 4 | 3 | 2 | 22.5 |
| | Example 5 | 0.29 | 33.3 | 1 | 3 | 2 | 2 | 78.1 |
| | Example 6 | 0.52 | 34.0 | 2 | 4 | 2 | 2 | 13.4 |
| | Example 7 | 0.47 | 37.0 | 3 | 4 | 2 | 2 | 14.8 |
| | Example 8 | 0.51 | 37.0 | 3 | 4 | 2 | 2 | 13.4 |
| | Comparative Example 1 | 1.70 | 29.7 | 25 | 1 | 1 | 1 | 9.5 |
| | Comparative Example 2 | 0.79 | 33.3 | 5 | 1 | 1 | 1 | 10.4 |
| | Comparative Example 3 | 0.73 | 26.6 | 25 | 3 | 1 | 1 | 8.9 |
| | Comparative Example 4 | 0.70 | 22.7 | 18 | 4 | 1 | 0 | 2.8 |
| | Comparative Example 5 | 0.58 | 27.6 | 19 | 3 | 1 | 1 | 6.5 |
| | Comparative Example 6 | 0.75 | 33.4 | 8 | 3 | 1 | 1 | 0.2 |
| | Comparative Example 7 | 0.10 | 25.7 | 0 | 3 | 1 | 1 | 13.2 |
| | Comparative Example 8 | 0.85 | 43.2 | 16 | 3 | 1 | 1 | 2.5 |
| | Comparative Example 9 | 0.66 | 22.7 | 18 | 3 | 1 | 1 | 2.8 |

The results in Table 3 show that good dispersion of the PGA particles makes the film less soluble and helps retain the shape of the film in a living body.

The film 15 (Comparative Example 7) with the amount of the PGA particles of 0.9 parts by weight per 100 parts by weight of the crosslinked polymer material was prone to disintegration despite the good dispersibility of the PGA particles. On the other hand, for the films 1 to 8 (Examples 1 to 8) with a small coefficient of variation CV of the number of particles contained in the rectangular regions despite the amount of the particles of 2 parts by weight or more, the disintegration of the film was delayed, and the disintegration of the film was suppressed. In addition, the films 12 to 14 and 17 (Comparative Examples 4 to 6 and 9) containing the biodegradable polyester particles having no constituent unit derived from polyglycolic acid or having less than 40 mass% of a constituent unit derived from polyglycolic acid had low dispersibility of the particles and were prone to disintegration. Furthermore, the films 9 to 11 (Comparative Examples 1 to 3) with a large coefficient of variation CV of the number of particles contained in the rectangular regions despite containing the PGA particles were also prone to disintegration.

The results in Table 3 show that the films with good dispersibility of the PGA particles have high tensile strength even in a wet state with water. A medical film with high tensile strength even in a wet state with water is less prone to tearing in a living body or the like and is easy to use in a living body.

The present application claims priority to JP 2023-077268 filed on May 9, 2023. The matters described in the specification, claims, and drawings at the time of the initial patent filing of the application are incorporated by reference into the present application.

[Industrial Applicability]

The medical film according to an embodiment of the present invention can retain its shape for a long period in a living body.

**Claims**

1. A medical film comprising:

   a crosslinked polymer material; and
   polyglycolic acid dispersoids that are particles containing a polymer having a constituent unit derived from glycolic acid or aggregates of the particles, wherein
   the polymer containing glycolic acid in a constituent unit is a polymer having a ratio of the constituent unit derived from glycolic acid to a total mass of the polymer of 40 mass% or more and 100 mass% or less,
   a content of the polyglycolic acid dispersoids per 100 parts by weight of the crosslinked polymer material is 2 parts by weight or more and 40 parts by weight or less, and
   a coefficient of variation CV of the number of particles contained in rectangular regions is 0.6 or less, where the coefficient of variation CV is determined from the number of particles measured in each of 16 rectangular regions with a size of 5 mm x 4 mm set so as not to overlap with each other on a surface of the medical film.

2. The medical film according to claim 1, wherein in the 16 rectangular regions, an average value of the number of aggregates with a diameter that is 5 or more times an average diameter of the polyglycolic acid dispersoids contained in the rectangular regions is 3 or less.

3. The medical film according to claim 1 or 2, wherein the polymer has a number-average molecular weight of 10000 or more and 150000 or less.

4. The medical film according to any one of claims 1 to 3, wherein the medical film has a tensile strength of 11 MPa or more and 100 MPa or less.

5. The medical film according to any one of claims 1 to 4, being an adhesion barrier material.

6. A method for producing a medical film, the method comprising:

   (a) mixing a solution or dispersion containing a crosslinked polymer material with particles containing a polymer derived from polyglycolic acid to form a composite of the crosslinked polymer material and the particles; and
   (b) forming the composite of the crosslinked polymer material and the particles into a film, wherein
   the polymer containing glycolic acid in a constituent unit is a polymer having a ratio of the constituent unit derived from glycolic acid to a total mass of the polymer of 40 mass% or more and 100 mass% or less,
   a viscosity of the solution or dispersion containing the crosslinked polymer material in step (a) is 2000 mPa·s or more, the viscosity being measured at 25°C and 10 rpm, and
   a number-average molecular weight of the polymer in step (a) is 15000 or more and 170000 or less.

FIG. 1B

FIG. 1A

## INTERNATIONAL SEARCH REPORT

| International application No. |
|---|
| **PCT/JP2024/017274** |

### A. CLASSIFICATION OF SUBJECT MATTER

*A61L 31/12*(2006.01)i; *A61L 27/20*(2006.01)i; *A61L 27/22*(2006.01)i; *A61L 27/26*(2006.01)i; *A61L 27/48*(2006.01)i; *A61L 27/58*(2006.01)i; *A61L 31/04*(2006.01)i; *A61L 31/06*(2006.01)i; *A61L 31/14*(2006.01)i; *C08J 5/18*(2006.01)i; *C08L 67/04*(2006.01)i; *C08L 101/00*(2006.01)i; *C08L 101/16*(2006.01)i

FI: A61L31/12 110; A61L27/20; A61L27/22; A61L27/26; A61L27/48; A61L27/58; A61L31/04 100; A61L31/04 120; A61L31/06; A61L31/14 500; C08J5/18; C08L67/04; C08L101/00 ZBP; C08L101/16

According to International Patent Classification (IPC) or to both national classification and IPC

### B. FIELDS SEARCHED

Minimum documentation searched (classification system followed by classification symbols)

A61L15/00-15/64; A61K9/00-9/72; A61K47/00-47/69; C08J5/00-5/02; C08J5/12-5/22; C08K3/00-13/08; C08L1/00-101/14; C08L101/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2021/201150 A1 (KUREHA CORPORATION) 07 October 2021 (2021-10-07) claims 1, 3, 6-9, paragraphs [0011], [0022], [0027], [0030], [0031], [0042], [0057], [0059], [0065], [0073], [0085], [0088] | 1-6 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **21 June 2024** | **16 July 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

## INTERNATIONAL SEARCH REPORT
### Information on patent family members

International application No.

**PCT/JP2024/017274**

| Patent document cited in search report | Publication date (day/month/year) | Patent family member(s) | Publication date (day/month/year) |
|---|---|---|---|
| WO 2021/201150 A1 | 07 October 2021 | US 2023/0119326 A1 claims 1, 3, 6-9, paragraphs [0042], [0044], [0047], [0048], [0059], [0074], [0076], [0082], [0098], [0110], [0113], [0116] | |

Form PCT/ISA/210 (patent family annex) (July 2022)

**REFERENCES CITED IN THE DESCRIPTION**

**Patent documents cited in the description**

- WO 2021201150 A
- RE 233005 B

- JP 2023077268 A